Europäisches Patentamt

European Patent Office

Office européen des brevets

(11) Veröffentlichungsnummer: **0 428 998 A1**

# (12) EUROPÄISCHE PATENTANMELDUNG

(21) Anmeldenummer: **90121837.0**

(22) Anmeldetag: **15.11.90**

(51) Int. Cl.5: **A61B 17/22**

(30) Priorität: **22.11.89 DE 8913770 U**

(43) Veröffentlichungstag der Anmeldung:
**29.05.91 Patentblatt 91/22**

(84) Benannte Vertragsstaaten:
**AT BE CH DE DK ES FR GB GR IT LI LU NL SE**

(71) Anmelder: **ANGIOMED AG**
**Wachhausstrasse 6**
**W-7500 Karlsruhe 41(DE)**

(72) Erfinder: **Schnepp-Pesch, Wolfram**

**Schönblick 6**
**W-7505 Ettlingen(DE)**
Erfinder: **Lindenberg, Josef**
**Käthe-Kollwitz-Strasse 10a**
**W-7500 Karlsruhe(DE)**

(74) Vertreter: **Lempert, Jost, Dipl.-Phys. Dr.**
**Patentanwälte Dipl.-Ing. Heiner Lichti**
**Dipl.-Phys. Dr. Jost Lempert Durlacher**
**Strasse 31 Postfach 410760**
**W-7500 Karlsruhe 41(DE)**

(54) **Vorrichtung zum Entfernen von Nieren- und Harnleitersteinen oder dergleichen.**

(57) Bei einer Vorrichtung (1) zum Entfernen von Nieren- und Harnleitersteinen oder dergleichen mit einer aus mehreren Einzeldrähten (4) gewundenen Litze (2) und einem an einem Ende der Litze (2) angeordneten Körbchen (9) zum Einfangen der Steine in Form mehrerer einen Käfig (9) bildender Drahtabschnitte (4) wurden die das Körbchen (9) bildenden Drahtabschnitte (4) erfindungsgemäß jeweils einstückig mit einem Einzeldraht (4) der Litze (2) ausgebildet.

## VORRICHTUNG ZUM ENTFERNEN VON NIEREN- UND HARNLEITERSTEINEN ODER DERGLEICHEN

Die Erfindung betrifft eine Vorrichtung zum Entfernen von Nieren-und Harnleitersteinen oder dergleichen mit einer aus mehreren Einzeldrähten gewundenen Litze und einem an einem Ende der Litze angeordneten Körbchen zum Einfangen der Steine in Form mehrerer einen Käfig bildender Drahtabschnitte.

Es sind Vorrichtungen zum Entfernen von Nieren- und Harnleitersteinen bekannt, bei denen an einem Ende, nämlich dem zum Arzt oder Operateur distalen Ende mit der vorgefertigten Litze ein separates Steinfangkörbchen derart verbunden ist, daß die der Litze zugewandten Enden von dieses bildenden Drahtabschnitten über das Ende der Litze geführt sind und über beides eine Hülse geschoben ist, die mit den Enden der Drahtabschnitte und mit den Enden der Litze fest verbunden ist, beispielsweise durch Quetschen und Verlöten. In gleicher Weise sind die der Litze abgewandten Enden der das Körbchen bildenden Drahtabschnitte wieder zusammengeführt. Zwischen den einen endlichen Abstand aufweisenden Drahtabschnitten können Steine eingefangen und mit der so erläuterten Vorrichtung dann entfernt werden. Es kann vorkommen, daß die Steine, beispielsweise weil sie zu groß sind, auf dem beschriebenen Weg nicht entfernt werden können. In diesen Fällen kann ein derartiger endogener Eingriff nicht vorgenommen werden oder muß abgebrochen werden und es ist eine Operation erforderlich. Zur Verkleinerung der Steine wurden schon aufwendige Lithotripter vorgeschlagen, mittels derer größere Steine durch Ultraschall zerstört werden, so daß die Bruchstücke auf natürlichem Wege oder der vorbeschriebenen Weise entfernt werden können. Lithotripter sind teuer und aufwendig.

Der Erfindung liegt die Aufgabe zugrunde eine Vorrichtung zum Entfernen von Nieren- und Harnleitersteinen zu schaffen, mit der ohne großen Aufwand und teuren Vorrichtung auch eine Zerkleinerung und damit Entfernung von größeren Steinen möglich ist.

Zur Lösung der genannten Aufgabe sieht die Erfindung vor, daß die das Körbchen bildenden Drahtabschnitte jeweils einstückig mit einem Einzeldraht der Litze ausgebildet sind.

Die erfindungsgemäße Vorrichtung mit einstückig mit der Litze ausgebildetem Steinfangkörbchen wird derart eingesetzt, daß nach Einbringen zu dem Ort, an dem sich die Steine befinden, beispielsweise im oberen Bereich des Harnleiters oder der Niere und Einfangen eines solchen Steins im Steinfangkörbchen dieses in einem Gegenhalterohr eingezogen wird, so daß der Stein zunächst sicher im Körbchen fixiert wird und anschließend ruckartig das Körbchen weiterhin in das Rohr eingezogen wird, das einen festen Durchmesser zumindestens an seinem vorderen dem Körbchen zugewandten Ende aufweisen muß. Unter der ausgeübten Zugkraft üben die Einzeldrahtabschnitte des Stein fangkörbchens eine radiale Kraft auf den zwischen ihnen eingefangenen Stein aus und zerbrechen oder zerschneiden diesen. Grundsätzlich könnte die derart beschriebene Steinzerstörung auch mit bekannten Vorrichtungen durchgeführt werden, bei denen Körbchen und Litze separate miteinander verbundene Teile sind. Es hat sich aber bei Einsatz derartiger bekannter Steinfangkörbchen herausgestellt, daß die Verbindungsstelle zwischen Körbchen und Litze eine "Sollbruchstelle" bildet und bei vielen, insbesondere harten Steinen nicht diese zerstört werden, sondern vielmehr unter der ausgeübten Kraft das Körbchen beschädigt wird, indem die Enden der Einzeldrahtabschnitte von der Litze bzw. der beide umfassenden Hülse abgerissen werden. Dies hat nicht nur den Nachteil, daß der Stein nicht entfernt werden kann, sondern vielmehr das Körbchen mit Litze, soweit einzelnes noch durch einzelne Drähte gehalten wird, herausgezogen und ein erneuter Eingriff vorgenommen werden muß. Es besteht auch die Gefahr, daß die abgerissenen Enden der Drahtabschnitte des Körbchens zu Beschädigungen des Gewebes führen können. Darüber hinaus muß gegebenenfalls ein vollständig abgerissenes Körbchen operativ entfernt werden. Diese den Einsatz bekannter Steinfangkörbchen problematisch und gefährlich machende Umstände zur Zerstörung von Steinen werden durch die erfindungsgemäße Ausgestaltung der Vorrichtung zum Entfernen solcher Steine vollständig vermieden. Es ist daher insbesondere sinnvoll auch zum Entfernen von Steinen in üblicherweise also alleine durch Einfangen die erfindungsgemäße Vorrichtung einzusetzen, so daß in dem Falle, daß sich nachträglich herausstellt, daß ein Stein zunächst zerkleinert oder zerstört werden muß, nicht die ursprünglich eingesetzte (herkömmliche) Steinfangvorrichtung zunächst herausgenommen und eine erfindungsgemäße eingeführt werden muß, um ein zuverlässiges Zerstören der Steine zu bewirken.

In bevorzugter Ausgestaltung ist weiterhin vorgesehen, daß die freien Enden der das Körbchen bildenden Drahtabschnitte durch eine Hülse zusammengehalten werden. Eine Weiterbildung sieht vor, daß die Enden der Drahtabschnitte mit der Hülse verlötet sind In weiterer Ausbildung kann darüber hinaus vorgesehen sein, daß im Übergangsbereich zwischen Körbchen und Litze eine Hülse angeordnet ist, wobei insbesondere die Hülse mit den die Litze bildenden Einzeldrähten verlötet ist.

Während herkömmlicherweise die Litze von hierauf spezialisierten Herstellern und Zulieferern bezogen wird und anschließend das Steinfangkörbchen mit dieser verbunden wird, ist zur Herstellung der erfindungsgemäßen Vorrichtung vorteilhafterweise nicht die fertige Litze das Ausgangsmaterial, sondern es werden vielmehr mehrere Einzeldrähte, vorzugsweise vier bis acht gebündelt. Anschließend werden die nebeneinander liegenden Drähte in einem vorgegebenen Längsabstand, innerhalb dessen das Körbchen gebildet werden soll, fest miteinander verbunden, beispielsweise durch Löten oder durch Aufsetzen der erwähnten Hülsen, Verquetschen und vorzugsweise ebenfalls Verlöten derselben mit den Einzeldrähten. Sodann können in dem nicht durch die Einzeldrähte durch die Hülsen eingefaßten Bereich der Einzeldrähte durch Verdrillen derselben die gewünschte Litze gebildet werden. Anschließend werden in dem abgeteilten Bereich die Einzeldrähte voneinander fort auseinandergebogen, um so das gewünschte Steinfangkörbchen zu bilden. Die beiden letzten Schritte können gegebenenfalls auch in umgekehrter Reihenfolge durchgeführt werden.

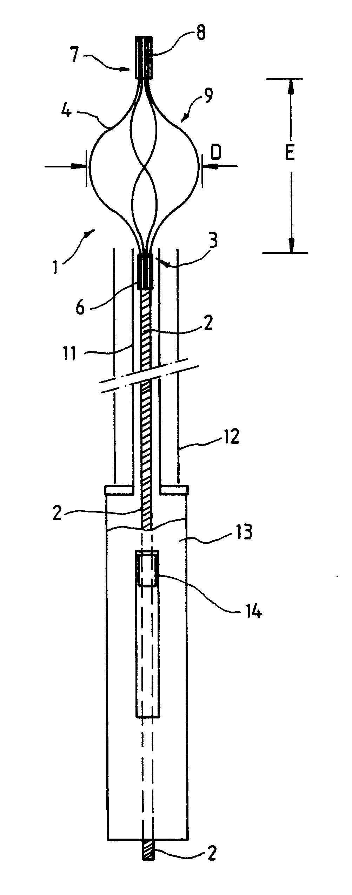

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im einzelnen erläutert sind. Dabei zeigt die einzige Figur
eine erfindungsgemäße Vorrichtung zum Entfernen von Nieren- und Harnleitersteinen oder dergleichen in Seitenansicht im Zusammenhang mit einer mit ihr verwendeten Vorrichtung zum Zerstören derartiger Steine.

Die erfindungsgemäße Vorrichtung 1 zum Entfernen von Nieren-und Harnleitersteinen oder dergleichen weist eine lange Litze 2 aus ineinander gewundenen Einzeldrähten auf. Die Einzeldrähte lösen sich bei 3 voneinander bis auf einen Abstand D, der ein Vielfaches der Litzenstärke beträgt. Am Ende der Litze 2, bevor die Einzeldrähte 4 sich voneinander lösen ist auf der Litze eine Hülse 6 aufgesetzt und mit der Litze fest verbunden, durch Quetschung oder vorzugsweise Verlötungen.

In einem endlichen Abstand E von der Hülse 6 sind die voneinander gelösten Einzeldrähte 4 wieder bei 3 und 7 eng zusammengeführt und über die Einzeldrähte ist dort ebenfalls eine Hülse 8 geschoben und fest mit den Einzeldrähten verbunden, ebenfalls durch Verquetschen oder vorzugsweise Verlöten. Zwischen den beiden Bereichen 3, 7 bzw. den Hülsen 6, 8 bilden die Drahtabschnitte 4 der die Litze 2 bildenen Einzelabschnitte einen Käfig oder ein Steinfangkörbchen 9, wobei wesentlich ist, daß die Drahtabschnitte 4 des Steinfangkörbchens 9 einstückig mit einem Einzeldraht der Drahtlitze 2 ausgebildet sind.

Weiterhin ist in der Zeichnung ein die Litze umgebendes Gegenhalterohr 11 dargestellt, das bei gewisser angularer Flexibilität insbesondere einen festen nicht veränderlichen Durchmesser an seiner distalen Stirnseite (bei 3) aufweist, der beispielsweise durch einen Ring an einem aus einem Geflecht gebildeten Rohr 11 gegeben sein kann. Das Gegenhalterohr 11 ist mit einer Spann- und Zugvorrichtung 13 für die Litze 2 verbunden, die durch die Vorrichtung 13 hindurchgeführt und mit einem relativ zu deren Gehäuse beweglichen Teil derselben, das vorzugsweise unter Federspannung bringbar ist, verbunden ist.

Nachdem zunächst der Katheder 12 gelegt wurde, wird zum Einsatz die Litze 2 mit dem an deren relativ zum Arzt gesehen distalen Ende ausgebildeten Steinfangkörbchen 9 vorzugsweise zusammen mit dem Gegenhalterohr 11 bis in den Bereich hindurchgeschoben, in dem sich die einzufangenden Steine befinden. Diese werden dann in üblicher Weise mit dem Steinfangkörbchen 9 eingefangen und in diesem dadurch eingespannt, daß die Litze relativ zum Gegenhalterohr 11 in dieses hineingezogen wird, wodurch sich das Steinfangkörbchen 9 verengt und verkürzt, so daß der Stein von diesem zuverlässig festgehalten wird. Anschließend kann, soweit es sich um einen Stein handelt, der nicht so groß ist, dieser in bekannter Weise durch den Katheder 12 entfernt werden.

Wenn nun aber ein eingefangener Stein zu groß ist, als daß er vollständig entfernt werden könnte, so wird nach Fixieren des Steins in der vorstehend beschriebenen Weise die Litze ruckartig weiter in und durch das Gegenhalterohr 11 gezogen, beispielsweise mittels einem unter einer Federwirkung stehenden Spannelement (bei 14 angedeutet) der Vorrichtung 13. Da dabei die das Körbchen 9 bildenden Drahtabschnitte ebenfalls weiter in das Gegenhalterohr 11 hineingezogen werden, dieses sich aber an seinem distalen Ende 13 nicht aufweiten kann, üben die Einzeldrahtabschnitte 4 eine Radialkraft auf den Stein aus und zerbrechen bzw. zerschneiden diesen in Einzelstücke, die dann teilweise natürlich abgehen können bzw. ausgespült werden oder aber es können größere Bruchstücke einzeln durch das Steinfangkörbchen 9 aufgenommen, eingefangen und in der oben beschriebenen Weise durch den Katheder 12 entfernt werden.

## Ansprüche

1. Vorrichtung zum Entfernen von Nieren- und Harnleitersteinen oder dergleichen mit einer aus mehreren Einzeldrähten gewundenen Litze und einem an einem Ende der Litze angeordneten Körbchen zum Einfangen der Steine in Form mehrerer einen Käfig bildender Drahtabschnitte, dadurch gekennzeichnet, daß die das Körbchen (9) bildenden Drahtabschnitte (4) jeweils einstückig mit einem Einzeldraht der Litze (2) ausgebildet sind.

2. Vorrichtung nach Anspruch 1, dadurch gekennzeichnet, daß die freien Enden der das Körbchen (9) bildenden Drahtabschnitte (4) durch eine Hülse (8) zusammengehalten werden.

3. Vorrichtung nach Anspruch 2, dadurch gekennzeichnet, daß die Enden der Drahtabschnitte (4) mit der Hülse (8) verlötet sind.

4. Vorrichtung nach einem der vorangehenden Ansprüche, dadurch gekennzeichnet, daß im Übergangsbereich zwischen Körbchen (9) und Litze (2) eine Hülse (6) angeordnet ist.

5. Vorrichtung nach Anspruch 4, dadurch gekennzeichnet, daß die Hülse (6) mit den die Litze (2) bildenden Einzeldrähten verlötet ist.

Europäisches
Patentamt

EUROPÄISCHER
RECHERCHENBERICHT

Nummer der Anmeldung

EP 90 12 1837

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 611 594 (GRAYHACK)<br>* Spalte 3, Zeilen 12-26; Spalte 3, Zeile 62 - Spalte 4, Zeile 5; Figuren 1,3-7,14,15,17,21 *<br>- - - | 1,2 | A 61 B 17/22 |
| Y | | 3-5 | |
| Y | EP-A-0 169 784 (SYNTHELABO)<br>* Seite 5, Zeilen 1-15; Figur 1 *<br>- - - | 3-5 | |
| A | | 1,2 | |
| X | FR-A-2 317 903 (A.H.S.C.)<br>* Seite 4, Zeilen 26-35; Seite 6, Zeile 23 - Seite 7, Zeile 25; Seite 7, Zeile 31 - Seite 8, Zeile 3; Figuren 1,4 *<br>- - - | 1,2,3-5 | |
| Y | DE-U-8 904 213 (GIP)<br>* Seite 4; Figur *<br>- - - | 3-5 | |
| A | | 1,2 | |
| A | DE-U-8 626 048 (PAULDRACH)<br>* Seite 3, Zeile 24 - Seite 4, Zeile 14; Figuren *<br>- - - | 1-5 | |
| A | GB-A-2 066 668 (PORGES)<br>- - - | | RECHERCHIERTE SACHGEBIETE (Int. Cl.5) |
| A | DE-A-3 343 231 (PAULDRACH)<br>- - - - - | | A 61 B |

Der vorliegende Recherchenbericht wurde für alle Patentansprüche erstellt

| Recherchenort | Abschlußdatum der Recherche | Prüfer |
|---|---|---|
| Den Haag | 15 Januar 91 | KLEIN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument